# EUROPEAN PATENT APPLICATION

(11) **EP 3 901 256 A1**
(43) Date of publication of application: **27.10.2021**
(21) Application number: 20170662.9
(22) Date of filing: 21.04.2020
(51) Int. Cl.: C12N 9/10, C12N 15/52, C12P 7/22, C12P 7/42

(54) **OPTIMIZED PRODUCTION OF CBGA FROM OLIVETOL ACID AND GERANYL PYROPHOSPHATE VIA SYNNPHB**

(71) Applicant: Synbionik GmbH, 60314 Frankfurt am Main (DE)
(72) Inventor: SCHMITT, Patrick, 55122 Mainz (DE); MACIEJKO, Jakob, 65239 Hochheim am Main (DE); SAITOV, Ali, 63067 Offenbach am Main (DE); DICK, Markus, 63067 Offenbach am Main (DE); TABOADA, Andrea, 60439 Frankfurt am Main (DE)
(74) Representative: Kilger, Ute

(57) **Abstract**

The prenyltransferase is a protein which catalyzes the prenylation of olivetolic acid (OA) with geranyl pyrophosphate (GPP) to produce CBGA. However, CBGA production using prenyltransferase NphB is extremely poor and generates the side-product 2-O-geranyl olivetolate. This invention describes the prenyltransferase SynNphB from the *Streptomyces sp.* and a set of mutations which enhances the CBGA production by enhancing the turnover rate and specificity of the enzyme while decreasing the yield of the side-production 2-O-geranyl olivetolate in microorganisms.

## Description

### Background

Prenyltransferases are a class of enzymes that transfer allylic prenyl groups. Prenyl transferases commonly refer to prenyl diphosphate synthases. Allylic prenyl groups are substituent with the structural formula H₂C=CH-CH₂R, where R is the rest of the molecule to acceptor molecules. For example, allyl alcohol (H₂C=CH-CH₂OH), allyl chloride (H₂C=CH-CH₂Cl), crotyl alcohol (CH₃CH=CH-CH₂OH, dimethylallyl pyrophosphate (central in the biosynthesis of terpenes and a precursor to many natural products, including natural rubber) and transitional metals, such as allylpalladium chloride dimer (Takahashi et al. 2006) (Maurer-Stroh et al. 2003).

Cannabinoids (e.g. cannabigerolic acid (CBGA)) are the active chemicals in medical marijuana and are similar to the chemicals inside the body involved in appetite, memory, movement and pain. Multiple research suggest that cannabinoids reduce anxiety, reduce inflammation, relieve pain, control nausea, vomiting caused by chemotherapy, kill cancer cells, slow tumor growth, relax tight muscles in people with multiple sclerosis, stimulate appetite and improve weight gain in people with cancer and AIDS (Bonn-Miller et al. 2019) (Kaur et al. 2016) (Schrot et al. 2016). Consequently, cannabinoids have become molecules of high interest for medical research and pharmacological practices.

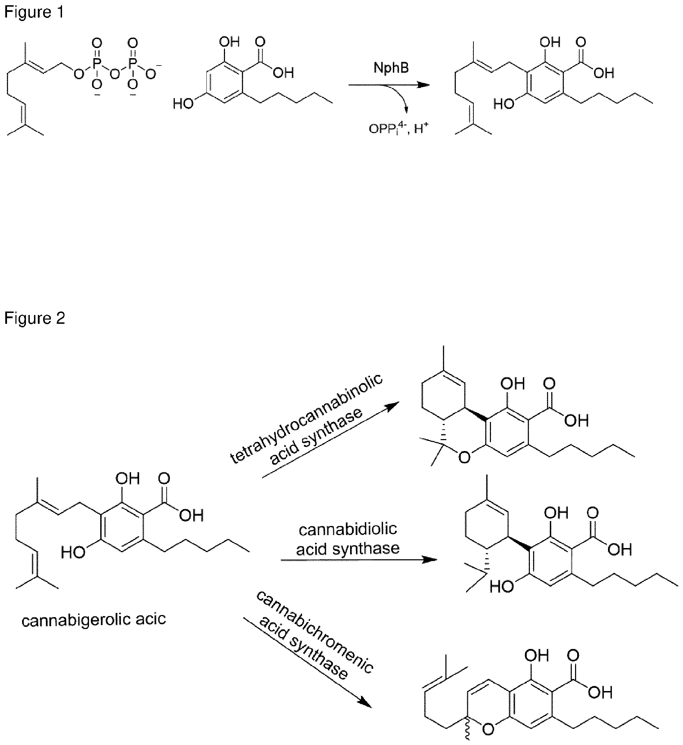

CBGA (cannabigerolic acid) is a foundational compound of the cannabis flower. CBGA is formed when olivetolic acid (OA) and geranyl pyrophosphate (GPP) combine. OA and GPP are two organic compounds natural contained in the cannabis plant. CBGA is converted to CBG (cannabigerol), THC (tetrahydrocannabinol) or CBD (cannabidiol) (see Figure 2). However, a research team in Dortmund University engineered a yeast to synthesize cannabinoids in yeast. They were able to achieve this by synthesizing CBGA from OA and GPP. From the synthetized CBGA they were able to produce THCA (tetrahydrocannabinolic acid) (Zirpel et al. 2017).

CBGA is the precursor molecule to a wide range of cannabinoids (see Figure 2). Since cannabinoids are becoming part of the mainstream subject matter of medical research and indicate to have high pharmacological potential, alternative routes for the high quantity and quality production of CBGA must be developed in order to supply a future high demand. Therefore, a method of enhancing the production of CBGA in the most efficient way (low yield of side products), with the highest standard of quality of production (Good Manufacturing Practices (G.M.P.)), able to be scalable, with lowest cost and environmental impact must be proposed in order to overcome this problem.

In the scientific article *A Cell-free Platform for the Prenylation of Natural Products and Application to Cannabinoid Production* it is described a flexible cell-free enzymatic prenylating system that generates isoprenyl pyrophosphate substrates from glucose to prenylate an array of natural products. The system provides an efficient route to cannabinoid precursors, CBGA, and cannabigerovarinic acid (CBGVA) at >1 g/L, and a single enzymatic step converts the precursors into CBDA and cannabidivarinic acid (CBDVA) (Valliere et al. 2019). However, this research does not propose a scalable nor cost efficient method. Since the proposed method requires the use of enzymes, this method would need periodical restoking and resetting of the enzymes after they are not able to work properly. Consequently, this raises the cost of production per batch of CBGA that it is produced. Additionally, personal must be trained in manipulation of these enzymes in order to reset the experimental setting for each batch. This will raise the cost of production and scalability would require high investment. Finally, this method describes the use of additional steps to remove side products making this method not efficient for high quantity production of CBGA in short time.

WO2017139496A1 describes compositions and methods for producing cannabinoids and cannabinoid precursors in genetic engineered microorganisms from a carbohydrate source. The genetically modified microorganism has increased production of cannabigerolic acid (CBGA) relative to a microorganism of the same species without the genetic modifications, which increase the expression of the HMG-CoA Reductase 1 (HMGR1), an isopentenyl-diphosphate delta isomerase 1 (IDI1), geranyl pyrophosphate synthase (GPPS), a farnesyl pyrophosphate synthase (FPPS), mutated farnesyl pyrophosphate synthase (mFPPS), olivetolate geranyltransferase (GOGT), or a combination thereof. However, this method does not propose a cost-efficient nor long-term solution. Due to the multiple exogenous genetic material that is required to be inserted into a microorganisms in order to produce CBGA, further experimental research will be required to ensure that the genetic engineered microorganism will not mutate nor loose, through multiple generations of the microorganism, the exogenic sequences MGR1, IDI1, GPPS, FPPS, mFPPS and GOGT. Additionally, multiple side products are produced making the yield of CBGA lower. Therefore, this method is not efficient in producing high quantities of CBGA in short periods of time.

WO2014159688A1 describes a method for the biosynthesis of CBGA, THCA, CBDA, CBCA, CBDVA, THCVA, CBGVA, and CBCVA utilizing genetic engineering by modifying the genes ERG, IDI, gal80p, upc2-1, HMGR, tHMGR, ALD6, DPP1, and ADH2, which result in: exogenous sterol mutations for the uptake of exogenous sterols, more permeable cell membrane for easy inward flow of exogenous materials while easy outward flow of our compounds of interest, and carbon flux manipulation and blocking of other pathways for many fold higher yields of cannabinoids in *S. Cerevisiae* and *P. Pastoris.* However, this method does not propose a cost-efficient nor long-term solution. Due to the multiple exogenous genetic material that is required to be inserted in order to produce CBGA, further experimental research will be required to ensure that the genetic engineered microorganism will not mutate nor loose, through multiple generations of the microorganism, the exogenic sequences ERG, IDI, gal80p, upc2-1, HMGR, tHMGR, ALD6, DPP1, and ADH2. Additionally, multiple side products are produced making the yield of CBGA lower. Therefore, this method is not efficient in producing high quantities of CBGA.

### Brief Description of the Invention

The invention proposed describes a non-naturally occurring protein sequence, a prenyltransferase, able to enhance the production of CBGA and reduced the yield of the side product 2-O-geranyl olivetolate in microorganisms. SynNphB is achieved by inserting double (SynNphB-M1 and SynNphB-M2) and triple (SynNphB-M3, SynNphB-M4, SynNphB-M5 and SynNphB-M6) mutations to the wild type Prenyltransferase NphB ((WP_078582630.1 Gl: 1154209950) from the *Streptomyces species.* These mutations increase the turnover rate and reduce the side-production 2-O-geranyl olivetolate. SynNphB has the technical feature and advantage that it can be inserted into microorganisms such as yeasts and bacteria in order to enhance the production of CBGA, which then can further be used to produce further cannabinoids. This catalytic advantage allows the synthesis of the CBGA in the most efficient way with low yield of side products (2-O-geranyl olivetolate). Also, it ensures the highest standard of quality of production since it can have G.M.P. (Good Manufacturing Practices) standards. Additionally, it is able to be scalable due to the use of fast-growing microorganisms (yeast and bacteria), which can produce high quantities of CBGA without the need of restock or restart the batch. Therefore, this invention proposes a solution with low cost and environmental impact due the nature of the microorganisms and the lack of need of constant human intervention to achieve CBGA as a product.

### Description of the Invention

### 1. Desired Function of the Non-naturally Occurring Protein

A key step for cannabinoid biosynthesis is the connection of the components olivetolic acid (OA) and geranyl pyrophosphate (GPP) to generate the final product cannabigerolic acid (CBGA). This C-C bond formation is catalyzed by the prenyltransferase NphB (see Figure 1). The CBGA production through natural routes is extremely poor and generates the side-product 2-O-geranyl olivetolate. Additionally, CBGA Synthase from plants itself is a membrane protein and doesn't work well in bacteria. As such, protein engineering of NphB is needed to improve its activity and specificity towards the desired product CBGA. Additionally, NphB is not a membrane anchored protein meaning that it is hydrophilic and therefore it dissolves well in the cytosol (Wang Z. et al. 2017).

The non-naturally occurring protein sequence catalyzes the C-C bond formation (such as prenyltransferase NphB) the connection of the components olivetolic acid (OA) and geranyl pyrophosphate (GPP) to generate the final product cannabigerolic acid (CBGA). However, this non-naturally occurring protein sequence can be stably inserted into a microorganism's genome. In this way, the microorganism is able to produce high quantities of CBGA with low yield of side products.

The 3-D model of NphB features a α/β barrel fold termed PT-barrel, composed of 10 anti-parallel β-strands surround by 10-α helices. Inside the barrel, a spacious and solvent accessible binding pocket is provided and two substrates molecules GPP and OA are bound. GPP is stabilized by interactions between its negatively charged diphosphate moiety and several amino acid sidechains such as lysine, tyrosine and arginine, in addition to Mg2+ (Yang et al. 2012).

### 2. Wild Type Sequence (NphB)

In a preferred embodiment the wild type protein sequence NphB (WT) from the *Streptomyces species* (WP_078582630.1 GI: 1154209950) identified as SEQ ID_NO 1:

MSGAAEVERVYSAMEESAGLLDVACSREKIQPILTAFQDVLADGVIVFSMANGRHATELD
FSISVPAGHGDPYAAALEHGLIPATGHPVGDLLADTQKALPVSMFAVDGEVTSGFKKTYA
FFPTDDMPGLAQLIDIPSMPPSVAENAELFGRYGLDKVQMISLDYKKNQVNLYFSNLNPE
FLQPEPVQAMVREMGLQLPADKGLAFAKRSFAVYPTLSWDSAKIERLCFAVISTDPTLAP
AQEQADLDLFSTYANNAPYAYAGEKRTLVYGLTLSPSEEYYKLGSYYQISDIQRKLLKAF
DALTD

### 3. Double Mutations

The non-naturally occurring protein is modified to improve the CBGA production while having a low yield of side products.

A structural homologue of NphB has recently been characterized (Valliere et al. 2019). They identified beneficial single- and double-point mutations that increased the overall yield and reduced the side product formation.

Therefore, a two double point mutation was inserted into NphB: G284S-Y286A and A230S-Y286V was achieved by using QuikChange PCR (BMC Biotechnol. 2008). By inserting the following mutations, a high turnover rate achieved by the enzyme and reduced the side-production 2-O-geranyl olivetolate.

In one embodiment, the non-naturally occurring protein sequence is a double mutant (SynNphB-M1) wherein in the amino acid 284, a Glycine (G), of the wild type protein sequence (identified as SEQ ID_NO 1) has been modified to a Serine (S) (G284S) and in the amino acid 286, a Tyrosine (Y), of the wild type protein sequence (identified as SEQ ID_NO 1) has been modified to a Alanine (A) (Y286A). The resulting non-naturally occurring protein sequence is identified as SEQ ID_NO 2:

MSGAAEVERVYSAMEESAGLLDVACSREKIQPILTAFQDVLADGVIVFSMANGRHATELD
FSISVPAGHGDPYAAALEHGLIPATGHPVGDLLADTQKALPVSMFAVDGEVTSGFKKTYA
FFPTDDMPGLAQLIDIPSMPPSVAENAELFGRYGLDKVQMISLDYKKNQVNLYFSNLNPE
FLQPEPVQAMVREMGLQLPADKGLAFAKRSFAVYPTLSWDSAKIERLCFAVISTDPTLAP
AQEQADLDLFSTYANNAPYAYAGEKRTLVYGLTLSPSEEYYKLSSAYQISDIQRKLLKAF

DALTD

In one embodiment the present application relates to a non-naturally occurring protein modified to improve CBGA production with decreased yield of side products comprising the sequence SEQ ID_NO 2 or a protein sequence having at least 70% identity thereof, respectively.

In one embodiment the present application relates to a non-naturally occurring protein modified to improve CBGA production with decreased yield of side products comprising the sequence SEQ ID_NO 2 or a protein sequence having at least 80% identity thereof, respectively.

In one embodiment the present application relates to a non-naturally occurring protein modified to improve CBGA production with decreased yield of side products comprising the sequence SEQ ID_NO 2 or a protein sequence having at least 90% identity thereof, respectively.

In one embodiment, the non-naturally occurring protein sequence is a double mutant (SynNphB-M2) wherein in the amino acid 230, an Alanine (A), of the wild type protein sequence (identified as SEQ ID_NO 1) has been modified to a Serine (S) (A230S) and in the amino acid 286, a Tyrosine (Y), of the wild type protein sequence (identified as SEQ ID_NO 1) has been modified to an Alanine (A) (Y286A). The resulting non-naturally occurring protein sequence is identified as SEQ ID_NO 3:

MSGAAEVERVYSAMEESAGLLDVACSREKIQPILTAFQDVLADGVIVFSMANGRHATELD
FSISVPAGHGDPYAAALEHGLIPATGHPVGDLLADTQKALPVSMFAVDGEVTSGFKKTYA
FFPTDDMPGLAQLIDIPSMPPSVAENAELFGRYGLDKVQMISLDYKKNQVNLYFSNLNPE
FLQPEPVQAMVREMGLQLPADKGLAFAKRSFAVYPTLSWDSAKIERLCFSVISTDPTLAP
AQEQADLDLFSTYANNAPYAYAGEKRTLVYGLTLSPSEEYYKLGSAYQISDIQRKLLKAF
DALTD

In one embodiment the present application relates to a non-naturally occurring protein modified to improve CBGA production with decreased yield of side products comprising the sequence SEQ ID_NO 3 or a protein sequence having at least 70% identity thereof, respectively.

In one embodiment the present application relates to a non-naturally occurring protein modified to improve CBGA production with decreased yield of side products comprising the sequence SEQ ID_NO 3 or a protein sequence having at least 80% identity thereof, respectively.

In one embodiment the present application relates to a non-naturally occurring protein modified to improve CBGA production with decreased yield of side products comprising the sequence SEQ ID_NO 3 or a protein sequence having at least 90% identity thereof, respectively.

### 4. Triple Mutations

### a. Triple Mutation on SynNphB-M1

The best variant was then chosen for a second round of mutagenesis. Docking studies using the Glide-Software from Schroedinger (Friesner et al. 2006) suggested that mutations of D108 may have an additional positive effect on substrate specificity/activity. Hence, random site-directed mutagenesis was chosen to mutate position 108 to all 20 possible amino acids (Kille et al. 2013).

In one embodiment, a third mutation was inserted on the double mutant SynNphB-M1 to increase further the turnover rate of the enzyme and reduced the side-production 2-O-geranyl olivetolate. Through computational methods, it was found that the exchange of amino acid Aspartic acid (D) on the position 108 (D108) of the SynNphB-M1 (identified as SEQ ID_NO 2) to Tyrosine (Y), Leucine (L), Isoleucine (I) or Arginine (N) improves the enzyme activity and specificity furthermore over the double mutant SynNphB-M1 and WT.

In one embodiment, the non-naturally occurring protein sequence is a triple mutant (SynNphB-M3) wherein in the amino acid 108, a Aspartic acid (D), of the wild type protein sequence (identified as SEQ ID_NO 1) has been modified to a Tyrosine (Y) (D108Y), in the amino acid 284, a Glycine (G), of the wild type protein sequence (identified as SEQ ID_NO 1) has been modified to a Serine (S) (G284S) and in the amino acid 286, a Tyrosine (Y), of the wild type protein sequence (identified as SEQ ID_NO 1) has been modified to an Alanine (A) (Y286A). The resulting non-naturally occurring protein sequence is identified as SEQ ID_NO 4:

MSGAAEVERVYSAMEESAGLLDVACSREKIQPILTAFQDVLADGVIVFSMANGRHATELD
FSISVPAGHGDPYAAALEHGLIPATGHPVGDLLADTQKALPVSMFAVYGEVTSGFKKTYA
FFPTDDMPGLAQLIDIPSMPPSVAENAELFGRYGLDKVQMISLDYKKNQVNLYFSNLNPE
FLQPEPVQAMVREMGLQLPADKGLAFAKRSFAVYPTLSWDSAKIERLCFAVISTDPTLAP
AQEQADLDLFSTYANNAPYAYAGEKRTLVYGLTLSPSEEYYKLSSAYQISDIQRKLLKAF
DALTD

In one embodiment the present application relates to a non-naturally occurring protein modified to improve CBGA production with decreased yield of side products comprising the sequence SEQ ID_NO 4 or a protein sequence having at least 70% identity thereof, respectively.

In one embodiment the present application relates to a non-naturally occurring protein modified to improve CBGA production with decreased yield of side products comprising the sequence SEQ ID_NO 4 or a protein sequence having at least 80% identity thereof, respectively.

In one embodiment the present application relates to a non-naturally occurring protein modified to improve CBGA production with decreased yield of side products comprising the sequence SEQ ID_NO 4 or a protein sequence having at least 90% identity thereof, respectively.

In one embodiment, the non-naturally occurring protein sequence is a triple mutant (SynNphB-M4) wherein in the amino acid 108, a Aspartic acid (D), of the wild type protein sequence (identified as SEQ ID_NO 1) has been modified to a Leucine (L) (D108L), in the amino acid 284, a Glycine (G), of the wild type protein sequence (identified as SEQ ID_NO 1) has been modified to a Serine (S) (G284S) and in the amino acid 286, a Tyrosine (Y), of the wild type protein sequence (identified as SEQ ID_NO 1) has been modified to an Alanine (A) (Y286A). The resulting non-naturally occurring protein sequence is identified as identified as SEQ ID_NO 5:

MSGAAEVERVYSAMEESAGLLDVACSREKIQPILTAFQDVLADGVIVFSMANGRHATELD
FSISVPAGHGDPYAAALEHGLIPATGHPVGDLLADTQKALPVSMFAVLGEVTSGFKKTYA
FFPTDDMPGLAQLIDIPSMPPSVAENAELFGRYGLDKVQMISLDYKKNQVNLYFSNLNPE
FLQPEPVQAMVREMGLQLPADKGLAFAKRSFAVYPTLSWDSAKIERLCFAVISTDPTLAP
AQEQADLDLFSTYANNAPYAYAGEKRTLVYGLTLSPSEEYYKLSSAYQISDIQRKLLKAF
DALTD

In one embodiment the present application relates to a non-naturally occurring protein modified to improve CBGA production with decreased yield of side products comprising the sequence SEQ ID_NO 5 or a protein sequence having at least 70% identity thereof, respectively.

In one embodiment the present application relates to a non-naturally occurring protein modified to improve CBGA production with decreased yield of side products comprising the sequence SEQ ID_NO 5 or a protein sequence having at least 80% identity thereof, respectively.

In one embodiment the present application relates to a non-naturally occurring protein modified to improve CBGA production with decreased yield of side products comprising the sequence SEQ ID_NO 5 or a protein sequence having at least 90% identity thereof, respectively.

In one embodiment, the non-naturally occurring protein sequence is a triple mutant (SynNphB-M5) wherein in the amino acid 108, a Aspartic acid (D), of the wild type protein sequence (identified as SEQ ID_NO 1) has been modified to a Isoleucine (I) (D108I), in the amino acid 284, a Glycine (G), of the wild type protein sequence (identified as SEQ ID_NO 1) has been modified to a Serine (S) (G284S) and in the amino acid 286, a Tyrosine (Y), of the wild type protein sequence (identified as SEQ ID_NO 1) has been modified to an Alanine (A) (Y286A). The resulting non-naturally occurring protein sequence is identified as SEQ ID_NO 6:

MSGAAEVERVYSAMEESAGLLDVACSREKIQPILTAFQDVLADGVIVFSMANGRHATELD
FSISVPAGHGDPYAAALEHGLIPATGHPVGDLLADTQKALPVSMFAVIGEVTSGFKKTYA
FFPTDDMPGLAQLIDIPSMPPSVAENAELFGRYGLDKVQMISLDYKKNQVNLYFSNLNPE
FLQPEPVQAMVREMGLQLPADKGLAFAKRSFAVYPTLSWDSAKIERLCFAVISTDPTLAP
AQEQADLDLFSTYANNAPYAYAGEKRTLVYGLTLSPSEEYYKLSSAYQISDIQRKLLKAF
DALTD

In one embodiment the present application relates to a non-naturally occurring protein modified to improve CBGA production with decreased yield of side products comprising the sequence SEQ ID_NO 6 or a protein sequence having at least 70% identity thereof, respectively.

In one embodiment the present application relates to a non-naturally occurring protein modified to improve CBGA production with decreased yield of side products comprising the sequence SEQ ID_NO 6 or a protein sequence having at least 80% identity thereof, respectively.

In one embodiment the present application relates to a non-naturally occurring protein modified to improve CBGA production with decreased yield of side products comprising the sequence SEQ ID_NO 6 or a protein sequence having at least 90% identity thereof, respectively.

In one embodiment, the non-naturally occurring protein sequence is a triple mutant (SynNphB-M6) wherein in the amino acid 108, a Aspartic acid (D), of the wild type protein sequence (identified as SEQ ID_NO 1) has been modified to a Arginine (N) (D108N), in the amino acid 284, a Glycine (G), of the wild type protein sequence (identified as SEQ ID_NO 1) has been modified to a Serine (S) (G284S) and in the amino acid 286, a Tyrosine (Y), of the wild type protein sequence (identified as SEQ ID_NO 1) has been modified to an Alanine (A) (Y286A). The resulting non-naturally occurring protein sequence is identified as SEQ ID_NO 7:

MSGAAEVERVYSAMEESAGLLDVACSREKIQPILTAFQDVLADGVIVFSMANGRHATELD
FSISVPAGHGDPYAAALEHGLIPATGHPVGDLLADTQKALPVSMFAVNGEVTSGFKKTYA

FFPTDDMPGLAQLIDIPSMPPSVAENAELFGRYGLDKVQMISLDYKKNQVNLYFSNLNPE
FLQPEPVQAMVREMGLQLPADKGLAFAKRSFAVYPTLSWDSAKIERLCFAVISTDPTLAP
AQEQADLDLFSTYANNAPYAYAGEKRTLVYGLTLSPSEEYYKLSSAYQISDIQRKLLKAF
DALTD

In one embodiment the present application relates to a non-naturally occurring protein modified to improve CBGA production with decreased yield of side products comprising the sequence SEQ ID_NO 7 or a protein sequence having at least 70% identity thereof, respectively.

In one embodiment the present application relates to a non-naturally occurring protein modified to improve CBGA production with decreased yield of side products comprising the sequence SEQ ID_NO 7 or a protein sequence having at least 80% identity thereof, respectively.

In one embodiment the present application relates to a non-naturally occurring protein modified to improve CBGA production with decreased yield of side products comprising the sequence SEQ ID_NO 7 or a protein sequence having at least 90% identity thereof, respectively.

### b. Triple Mutation on SynNphB-M2

The best variant was then chosen for a second round of mutagenesis. Docking studies using the Glide-Software from Schroedinger (Friesner et al. 2006) suggested that mutations of D108 may have an additional positive effect on substrate specificity/activity. Hence, random site-directed mutagenesis was chosen to mutate position 108 to all 20 possible amino acids (Kille et al. 2013).

In one embodiment, a third mutation was inserted on the double mutant SynNphB-M1 to increase further the turnover rate of the enzyme and reduced the side-production 2-O-geranyl olivetolate. Through computational methods, it was found that the exchange of amino acid Aspartic acid (D) on the position 108 (D108) of the SynNphB-M2 (identified as SEQ ID_NO 2) to Tyrosine (Y), Leucine (L), Isoleucine (I) or Arginine (N) improves the enzyme activity and specificity furthermore over the double mutant SynNphB-M2 and WT.

In one embodiment, the non-naturally occurring protein sequence is a triple mutant (SynNphB-M3) wherein in the amino acid 108, a Aspartic acid (D), of the wild type protein sequence (identified as SEQ ID_NO 1) has been modified to a Tyrosine (Y) (D108Y), in the amino acid 284, a Glycine (G), of the wild type protein sequence (identified as SEQ ID_NO 1) has been modified to a Serine (S) (G284S) and in the amino acid 286, a Tyrosine (Y), of the wild type protein sequence (identified as SEQ ID_NO 1) has been modified to an Alanine (A). The resulting non-naturally occurring protein sequence is identified as SEQ ID_NO 4:

MSGAAEVERVYSAMEESAGLLDVACSREKIQPILTAFQDVLADGVIVFSMANGRHATELD
FSISVPAGHGDPYAAALEHGLIPATGHPVGDLLADTQKALPVSMFAVYGEVTSGFKKTYA
FFPTDDMPGLAQLIDIPSMPPSVAENAELFGRYGLDKVQMISLDYKKNQVNLYFSNLNPE
FLQPEPVQAMVREMGLQLPADKGLAFAKRSFAVYPTLSWDSAKIERLCFAVISTDPTLAP
AQEQADLDLFSTYANNAPYAYAGEKRTLVYGLTLSPSEEYYKLSSAYQISDIQRKLLKAF
DALTD

In one embodiment the present application relates to a non-naturally occurring protein modified to improve CBGA production with decreased yield of side products comprising the sequence SEQ ID_NO 4 or a protein sequence having at least 70% identity thereof, respectively.

In one embodiment the present application relates to a non-naturally occurring protein modified to improve CBGA production with decreased yield of side products comprising the sequence SEQ ID_NO 4 or a protein sequence having at least 80% identity thereof, respectively.

In one embodiment the present application relates to a non-naturally occurring protein modified to improve CBGA production with decreased yield of side products comprising the sequence SEQ ID_NO 4 or a protein sequence having at least 90% identity thereof, respectively.

In one embodiment, the non-naturally occurring protein sequence is a triple mutant (SynNphB-M4) wherein in the amino acid 108, a Aspartic acid (D), of the wild type protein sequence (identified as SEQ ID_NO 1) has been modified to a Leucine (L) (D108L), in the amino acid 284, a Glycine (G), of the wild type protein sequence (identified as SEQ ID_NO 1) has been modified to a Serine (S) (G284S) and in the amino acid 286, a Tyrosine (Y), of the wild type protein sequence (identified as SEQ ID_NO 1) has been modified to an Alanine (A). The resulting non-naturally occurring protein sequence is identified as SEQ ID_NO 5:

MSGAAEVERVYSAMEESAGLLDVACSREKIQPILTAFQDVLADGVIVFSMANGRHATELD
FSISVPAGHGDPYAAALEHGLIPATGHPVGDLLADTQKALPVSMFAVLGEVTSGFKKTYA
FFPTDDMPGLAQLIDIPSMPPSVAENAELFGRYGLDKVQMISLDYKKNQVNLYFSNLNPE
FLQPEPVQAMVREMGLQLPADKGLAFAKRSFAVYPTLSWDSAKIERLCFAVISTDPTLAP
AQEQADLDLFSTYANNAPYAYAGEKRTLVYGLTLSPSEEYYKLSSAYQISDIQRKLLKAF
DALTD

In one embodiment the present application relates to a non-naturally occurring protein modified to improve CBGA production with decreased yield of side products comprising the sequence SEQ ID_NO 5 or a protein sequence having at least 70% identity thereof, respectively.

In one embodiment the present application relates to a non-naturally occurring protein modified to improve CBGA production with decreased yield of side products comprising the sequence SEQ ID_NO 5 or a protein sequence having at least 80% identity thereof, respectively.

In one embodiment the present application relates to a non-naturally occurring protein modified to improve CBGA production with decreased yield of side products comprising the sequence SEQ ID_NO 5 or a protein sequence having at least 90% identity thereof, respectively.

In one embodiment, the non-naturally occurring protein sequence is a triple mutant (SynNphB-M5) wherein in the amino acid 108, a Aspartic acid (D), of the wild type protein sequence (identified as SEQ ID_NO 1) has been modified to a Isoleucine (I) (D108I), in the amino acid 284, a Glycine (G), of the wild type protein sequence (identified as SEQ ID_NO 1) has been modified to a Serine (S) (G284S) and in the amino acid 286, a Tyrosine (Y), of the wild type protein sequence (identified as SEQ ID_NO 1) has been modified to an Alanine (A). The resulting non-naturally occurring protein sequence is identified as SEQ ID_NO 6:

MSGAAEVERVYSAMEESAGLLDVACSREKIQPILTAFQDVLADGVIVFSMANGRHATELD
FSISVPAGHGDPYAAALEHGLIPATGHPVGDLLADTQKALPVSMFAVIGEVTSGFKKTYA
FFPTDDMPGLAQLIDIPSMPPSVAENAELFGRYGLDKVQMISLDYKKNQVNLYFSNLNPE
FLQPEPVQAMVREMGLQLPADKGLAFAKRSFAVYPTLSWDSAKIERLCFAVISTDPTLAP
AQEQADLDLFSTYANNAPYAYAGEKRTLVYGLTLSPSEEYYKLSSAYQISDIQRKLLKAF
DALTD

In one embodiment the present application relates to a non-naturally occurring protein modified to improve CBGA production with decreased yield of side products comprising the sequence SEQ ID_NO 6 or a protein sequence having at least 70% identity thereof, respectively.

In one embodiment the present application relates to a non-naturally occurring protein modified to improve CBGA production with decreased yield of side products comprising the sequence SEQ ID_NO 6 or a protein sequence having at least 80% identity thereof, respectively.

In one embodiment the present application relates to a non-naturally occurring protein modified to improve CBGA production with decreased yield of side products comprising the sequence SEQ ID_NO 6 or a protein sequence having at least 90% identity thereof, respectively.

In one embodiment, the non-naturally occurring protein sequence is a triple mutant (SynNphB-M6) wherein in the amino acid 108, a Aspartic acid (D), of the wild type protein sequence (identified as SEQ ID_NO 1) has been modified to a Arginine (N) (D108N), in the amino acid 284, a Glycine (G), of the wild type protein sequence (identified as SEQ ID_NO 1) has been modified to a Serine (S) (G284S) and in the amino acid 286, a Tyrosine (Y), of the wild type protein sequence (identified as SEQ ID_NO 1) has been modified to an Alanine (A). The resulting non-naturally occurring protein sequence is identified as SEQ ID_NO 7:

MSGAAEVERVYSAMEESAGLLDVACSREKIQPILTAFQDVLADGVIVFSMANGRHATELD
FSISVPAGHGDPYAAALEHGLIPATGHPVGDLLADTQKALPVSMFAVNGEVTSGFKKTYA
FFPTDDMPGLAQLIDIPSMPPSVAENAELFGRYGLDKVQMISLDYKKNQVNLYFSNLNPE
FLQPEPVQAMVREMGLQLPADKGLAFAKRSFAVYPTLSWDSAKIERLCFAVISTDPTLAP
AQEQADLDLFSTYANNAPYAYAGEKRTLVYGLTLSPSEEYYKLSSAYQISDIQRKLLKAF
DALTD

In one embodiment the present application relates to a non-naturally occurring protein modified to improve CBGA production with decreased yield of side products comprising the sequence SEQ ID_NO 7 or a protein sequence having at least 70% identity thereof, respectively.

In one embodiment the present application relates to a non-naturally occurring protein modified to improve CBGA production with decreased yield of side products comprising the sequence SEQ ID_NO 7 or a protein sequence having at least 80% identity thereof, respectively.

In one embodiment the present application relates to a non-naturally occurring protein modified to improve CBGA production with decreased yield of side products comprising the sequence SEQ ID_NO 7 or a protein sequence having at least 90% identity thereof, respectively.

### 5. Introduction of the Non-naturally Occurring Protein into Microorganisms for the Enhanced Production of CBGA

### a. Microorganism Candidates for the Insertion of the Non-Naturally Occurring Protein

In one embodiment the microorganism for the insertion of the non-naturally occurring protein is selected from the genus *Escherichia, Saccharomyces, Clostridium, Bacillus, Lactococcus, Zymomonas, Corynebacterium, Pichia, Candida, Hansenula, Trichoderma, Acetobacterium, Ralstonia, Cupravidor, Salmonella, Klebsiella, Paenibacillus, Pseudomonas, Lactobacillus, Rhodococcus, Enterococcus, Alkaligenes, Brevibacterium, Methylobacterium, Methylococcus, Methylomonas, Methylocystis* and *Methylosinus.*

In a preferred embodiment, the microorganism for the insertion of the non-naturally occurring protein is selected from the group comprising of *Escherichia coli, Saccharomyces cerevisiae, Clostridium acetobutylicum, Clostridium beijerinckii, Clostridium saccharbutyricum, Clostridium saccharoperbutylacetonicum, Clostridium butyricum, Clostridium diolis, Clostridium kluyveri, Clostridium pasterianium, Clostridium novyi, Clostridium difficile, Clostridium thermocellum, Clostridium cellulolyticum, Clostridium cellulovorans, Clostridium phytofermentans, Lactococcus lactis, Bacillus subtilis, Bacillus licheniformis, Zymomonas mobilis, Klebsiella oxytoca, Klebsiella pneumonia, Corynebacterium glutamicum, Trichoderma reesei, Ralstonia eutropha, Cupriavidus necator, Pseudomonas putida, Lactobacillus plantarum* and *Methylob acterium extorquens.*

In another embodiment the microorganism for the insertion of the non-naturally occurring protein is selected from a group comprising a carboxydotrophic bacteria from the genus *Clostridium, Moorella, Oxobacter, Acetobacterium, Eubacterium* or *Butyribacterium.*

In one embodiment the microorganism for the insertion of the non-naturally occurring protein is a carboxydotrophic microorganism which is selected from a group comprising *Clostridium Ijungdahlii, Clostridium carboxydivorans, Clostridium ragsdalei, Clostridium autoethanogenum, Moorella thermoacetica, Moorella thermoautotrophica, Oxobacter pfennigii, Acetobacterium woodi, Eubacterium limosum* and *Butyribacterium methylotrophicum.*

In a preferred embodiment the microorganism is *Escherichia coli BLR (DE3) as it is used for the heterologous expression of unstable proteins or enzymes (Goffin et. al. 2017).*

In a preferred embodiment the microorganism is *Escherichia coli Rosetta2 (DE3)* as it is used for the heterologous expression of eukaryotic proteins or enzymes (Kopanic et. al. 2013).

In a preferred embodiment the microorganism is *Escherichia coli T7 Express* as it is an organism of choice for the general heterologous expression and production of recombinant proteins or enzymes (Lobstein et. al. 2012).

In a preferred embodiment the microorganism is *Escherichia coli BL21 (DE3)* as it is an organism of choice for the heterologous expression and production of recombinant proteins or enzymes (Rosano et al 2014).

In a preferred embodiment the microorganism is *Zymomonas mobilis* ZM4 as it contains a number of desirable characteristics such as the Entner-Doudoroff pathway and as a consequence makes it a suitable microorganism for metabolic engineering and production of bio-products (e.g. CBGA) (He et al 2014).

In a preferred embodiment the microorganism is *Saccharomyces Cerevisiae* as it is a eukaryotic microorganism and has advantages for the heterologous expression of eukaryotic (plant) enzymes. The robust cultivation and genetic modification of the microorganism is advantageous for metabolic engineering and expression of heterologous enzymes (Borgne 2012).

In a preferred embodiment the microorganism is *Clostridium autoethanogenum* as it is an anaerobic microorganism. Therefore, it can utilize an alternative carbon source. The carbon source for the fermentation reaction is a gaseous substrate containing at least one of CO, CO₂ and H₂. The substrate may be a waste gas obtained as a byproduct of an industrial process (for example steel manufacturing, gasification of biomass, coal, animal wastes, production of ferroalloys and municipal solid waste (Sun et al. 2019)), or from another source such as from automobile exhaust fumes (Xu et al. 2017).

### b. Genetic Modification of the Microorganism and Incorporation of the Non-naturally Occurring Protein into Microorganisms for the Enhanced Production of CBGA

The microorganism comprises one or more genes, which catalyze prenylation of olivetolic acid (OA) to produce cannabigerolic acid (CBGA) to improve CBGA production by enhancing the turnover rate and specificity for the enzyme.

The microorganism is genetically modified in a way that it comprises exogenous genetic material coding for the enzymatic step to form CBGA from olivetolic acid (OA) and geranylpyrophosphate (GPP).

Exogenous genetic material hereby defined DNA originating outside the organism of concern or study.

In one embodiment, the microorganism is modified to comprise SynNphB-M1 identified by SEQ ID_NO 2.

In one embodiment, the microorganism is modified to comprise SynNphB-M2 identified by SEQ ID_NO 3.

In one embodiment, the microorganism is modified to comprise SynNphB-M3 identified by SEQ ID_NO 4.

In one embodiment, the microorganism is modified to comprise SynNphB-M4 identified by SEQ ID_NO 5.

In one embodiment, the microorganism is modified to comprise SynNphB-M5 identified by SEQ ID_NO 6.

In one embodiment, the microorganism is modified to comprise SynNphB-M6 identified by SEQ ID_NO 7.

### c. Genetic Modification of Escherichia coli and Incorporation of the Non-naturally Occurring Protein into Microorganisms for the Enhanced Production of CBGA

In a one embodiment the microorganism is *Escherichia coli* and it is modified using genetic engineering tools to insert SynNphB-M1 identified by SEQ ID_NO 2.

In a one embodiment the microorganism is *Escherichia coli* and it is modified using genetic engineering tools to insert SynNphB-M2 identified by SEQ ID_NO 3.

In a one embodiment the microorganism is *Escherichia coli* and it is modified using genetic engineering tools to insert SynNphB-M3 identified by SEQ ID_NO 4.

In a one embodiment the microorganism is *Escherichia coli* and it is modified using genetic engineering tools to insert SynNphB-M4 identified by SEQ ID_NO 5.

In a one embodiment the microorganism is *Escherichia coli* and it is modified using genetic engineering tools to insert SynNphB-M5 identified by SEQ ID_NO 6.

In a preferred embodiment the microorganism is *Escherichia coli* and it is modified using genetic engineering tools to insert SynNphB-M6 identified by SEQ ID_NO 7.

### Genome Modification of Escherichia coli using CRISPR-Cas system

In an embodiment, *Escherichia coli* is modified genetically via the CRISPR-Cas system. This technique enables deletions, insertions or point mutations in the genomic DNA of the microorganism. The system can recognize a protospacer adjacent motif (PAM) next to the genomic target site and it generates a double-strand break. This is followed by DNA repair initiation through homologous directed repair if an exogenous homologous donor sequence is present (Zhao et. al. 2016). If the exogenous homologous donor sequence flanks the desired gene sequence, this sequence is stably introduced into the *Escherichia coli* genome.

In an embodiment, *Escherichia coli* can be modified genetically via the CRISPR-Cas system. Plasmids containing a Cas protein and a corresponding guide RNA are transformed together with the gene sequence flanked with the DNA fragments complementary to the insertion site. The plasmids are transformed by electroporation and subsequently spread on agar plates, supplemented with antibiotics. By colony PCR, the target sequence is amplified with the help of the corresponding primers and individual positive colonies are then identified with agarose gel electrophoresis and sequencing.

### Genome Modification of Escherichia coli via Lamba Red

In an embodiment, *Escherichia coli* is modified genetically via the phage Lambda-derived Red recombination system. This technique enables deletions, insertions or point mutations in the genomic DNA of *Escherichia coli.*

In an embodiment, a plasmid is used comprising the bacteriophage lambda red components. The repair template essential for the homologous recombination is designed to have homologous arms to the genomic DNA of the microorganism where the mutation is incorporated as well as containing the desired mutation. If the homologous arms flank the desired gene sequence, this sequence is stably introduced into the *Escherichia coli* genome. Both the plasmid and the repair template are transformed into *Escherichia coli.*

In an embodiment, the Lambda-derived Red recombination plasmids are transformed by electroporation into *Escherichia coli* and subsequently spread on agar plates, supplemented with antibiotics. By colony PCR, the target sequence is amplified with assistance of the corresponding primers and individual positive colonies are then identified with agarose gel electrophoresis and sequencing.

### Genome Modification of Escherichia coli via Homologous Recombination (HR).

Homologous recombination is used as a method for the introduction of genetic material to make the non-natural occurring microorganism. The genetic material for the homologous recombination contains a high degree of homology to the genetic sequence of the microorganism. Through an endogenous in cell mechanism, the region with a high degree of homology gets built into the genome creating a non-naturally occurring microorganism. The homologous regions are designed in a way that they flank a specific sequence region. This sequence region gets incorporated into the genome of the bacteria through homologous recombination.

In an embodiment, homologous gene sequence flanking the gene by electroporation into the microorganism and subsequently spread on agar plates, which have been supplemented with antibiotics. By colony PCR, the target sequence is amplified with the help of the corresponding primers and individual positive colonies are then identified with agarose gel electrophoresis and sequencing.

### d. Genetic Modification of Zvmomonas mobilis ZM4 and Incorporation of the Non-naturally Occurring Protein into Microorganisms for the Enhanced Production of CBGA

In a one embodiment the microorganism is *Zymomonas mobilis ZM4* and it is modified using genetic engineering tools to insert SynNphB-M1 identified by SEQ ID_NO 2.

In a one embodiment the microorganism is *Zymomonas mobilis ZM4* and it is modified using genetic engineering tools to insert SynNphB-M2 identified by SEQ ID_NO 3.

In a one embodiment the microorganism is *Zymomonas mobilis ZM4* and it is modified using genetic engineering tools to insert SynNphB-M3 identified by SEQ ID_NO 4.

In a one embodiment the microorganism is *Zymomonas mobilis ZM4* and it is modified using genetic engineering tools to insert SynNphB-M4 identified by SEQ ID_NO 5.

In a one embodiment the microorganism is *Zymomonas mobilis ZM4* and it is modified using genetic engineering tools to insert SynNphB-M5 identified by SEQ ID_NO 6.

In a preferred embodiment the microorganism is *Zymomonas mobilis ZM4* and it is modified using genetic engineering tools to insert SynNphB-M6 identified by SEQ ID_NO 7.

### Genetic Modification of Zvmomonas mobilis ZM4 Using Plasmid Shuttle Vectors

In an embodiment the genes, which enable the expression of enzymes used for the enhanced production of CBGA while decreasing the yield of the side-production 2-O-geranyl olivetolate are transformed into *Zymomonas mobilis* ZM4 via a shuttle vector plasmid.

The shuttle vector plasmid can contain one or more specific promoters, one or more multiple cloning sites for the insertion of single genes or gene clusters, one or more terminators, one or more resistance cassettes (selected from ampicillin resistance (AmpR), kanamycin resistance (KanaR), chloramphenicol resistance (CamR), spectinomycin resistance (SpecR), tetracycline resistance (TetR)) and an *Zymomonas mobilis* Origin of Replication (OR).

In an embodiment, the shuttle vectors with the incorporated genes are transformed into *Zymomonas mobilis* via electroporation and subsequently plated on agar plates containing one or more of the following antibiotics ampicillin, kanamycin, chloramphenicol, tetracycline and spectinomycin. Detection of the successful transformation of gene cassettes is accomplished via PCR and sequencing. The gene transcription and enzyme translation and activity are determined by transcriptome, proteome and metabolome analysis.

### Genome Modification of Zymomonas mobilis ZM4 Using CRISPR-Cas system

In an embodiment, the microorganism can be modified genetically via the CRISPR-Cas system. This technique enables deletions, insertions or point mutations in the genomic DNA of the microorganism.

In an embodiment the gram-negative bacterial strain *Zymomonas mobilis* is a genetically modified microorganism via the use of the endogenous CRISPR-Cas system (type III) in order to introduce deletions, insertions or point mutations in the genomic DNA of the microorganism (Zheng et. al. 2019).

### Genome Modification of Zymomonas mobilis ZM4 via Lamba Red

*Zymomonas mobilis* is genetically modified via the phage Lambda-derived Red recombination system. This technique enables deletions, insertions or point mutations in the genomic DNA of *Zymomonas mobilis.*

In an embodiment, a plasmid is used comprising the bacteriophage lambda red components. The repair template essential for the homologous recombination is designed to have homologous arms to the genomic DNA of the microorganism where the mutation is incorporated as well as containing the desired mutation. If the homologous arms flank the desired gene sequence, this sequence is stably introduced into the *Zymomonas mobilis* genome. Both the plasmid and the repair template are transformed into *Zymomonas mobilis.*

In an embodiment, the Lambda-derived Red recombination plasmids are transformed by electroporation into the microorganism and subsequently spread on agar plates, which have been supplemented with antibiotics. By colony PCR, the target sequence is amplified with the help of the corresponding primers and individual positive colonies are then identified with agarose gel electrophoresis and sequencing.

### Genome Modification of Zymomonas mobilis ZM4 via Homologous Directed Repair (HDR).

Homologous directed repair is used as a method for the introduction of genetic material to make the non-natural occurring microorganism. The genetic material for the homologous directed repair contains a high degree of homology to the genetic sequence of the microorganism. Through an endogenous in cell mechanism the region with a high degree of homology gets built into the genome creating a non-naturally occurring microorganism. The homologous regions are designed in a way that they flank a specific sequence region. This sequence region gets incorporated into the genome of the bacteria through homologous recombination.

In an embodiment, the incorporated region contains FRT-sequences from *Saccharomyces cerevisiae.* The FRT sequence is used as a FLP-FRT site-specific recombination system. Gene fragments flanking two FRT sequences are incorporated by the *Saccharomyces cerevisiae* FLT gene coding for the FLP recombinase enzyme (Zou et al 2012).

### e. Genetic Modification of Saccharomyces Cerevisiae and Incorporation of the Non-naturally Occurring Protein into Microorganisms for the Enhanced Production of CBGA

In a one embodiment the microorganism is *Saccharomyces Cerevisiae* and it is modified using genetic engineering tools to insert SynNphB-M1 identified by SEQ ID_NO 2.

In a one embodiment the microorganism is *Saccharomyces Cerevisiae* and it is modified using genetic engineering tools to insert SynNphB-M2 identified by SEQ ID_NO 3.

In a one embodiment the microorganism is *Saccharomyces Cerevisiae* and it is modified using genetic engineering tools to insert SynNphB-M3 identified by SEQ ID_NO 4.

In a one embodiment the microorganism is *Saccharomyces Cerevisiae* and it is modified using genetic engineering tools to insert SynNphB-M4 identified by SEQ ID_NO 5.

In a one embodiment the microorganism is *Saccharomyces Cerevisiae* and it is modified using genetic engineering tools to insert SynNphB-M5 identified by SEQ ID_NO 6.

In a preferred embodiment the microorganism is *Saccharomyces Cerevisiae* and it is modified using genetic engineering tools to insert SynNphB-M6 identified by SEQ ID_NO 7.

### Genetic Modification of Saccharomyces Cerevisiae Using Plasmid Shuttle Vectors

In an embodiment, the genes which enable the expression of enzymes used for the production of CBGA and apigenin as a possible intermediate product are transformed into *Saccharomyces Cerevisiae* via a shuttle vector plasmid.

The shuttle vector plasmid comprises one or more specific *Saccharomyces Cerevisiae* promoters, one or more multiple cloning sites for the insertion of single genes or gene clusters, one or more terminators, one or more resistance cassettes (selected from ampicillin resistance (AmpR), kanamycin resistance (KanaR), chloramphenicol resistance (CamR), spectinomycin resistance (SpecR), tetracycline resistance (TetR)) and an *Saccharomyces Cerevisiae* Origin of Replication (OR).

In an embodiment, the shuttle vectors containing the sequence SynNphB-M1 identified by SEQ ID_NO 2, SynNphB-M2 identified by SEQ ID_NO 3, SynNphB-M3 identified by SEQ ID_NO 4, SynNphB-M4 identified by SEQ ID_NO 5, SynNphB-M5 identified by SEQ ID_NO 6 or SynNphB-M6 identified by SEQ ID_NO 7 are transformed via electroporation and subsequently plated on agar plates containing one or more of the following antibiotics ampicillin, kanamycin, chloramphenicol, tetracycline and spectinomycin. The detection of the successful transformation of gene cassettes is accomplished via PCR and sequencing. The gene transcription and enzyme translation and activity are determined by transcriptome, proteome and metabolome analysis.

### Genome Modification of Saccharomyces Cerevisiae Using CRISPR-Cas System

In an embodiment, *Saccharomyces Cerevisiae* can be modified genetically via the CRISPR-Cas system. This technique enables deletions, insertions or point mutations in the genomic DNA of the microorganism. The system is able to recognize a protospacer adjacent motif (PAM) next to the genomic target site and generate a double-strand break. This is followed by DNA repair initiation through homologous directed repair if an exogenous homologous donor sequence is present. If the exogenous homologous donor sequence flanks the desired gene sequence, this sequence is stably introduced into the *Saccharomyces Cerevisiae* genome.

### Genome Modification of Saccharomyces Cerevisiae via Homologous Directed Repair (HDR).

Homologous directed repair is used as a method for the introduction of genetic material to make the non-natural occurring bacterial. The genetic material for the homologous directed repair contains a high degree of homology to the genetic sequence of the microorganism. Through an endogenous in cell mechanism the region with a high degree of homology gets built into the genome creating a non-naturally occurring microorganism. The homologous regions are designed in a way that they flank a specific sequence region. This sequence region gets incorporated into the genome of the bacteria through homologous recombination.

### f. Genetic Modification of Clostridium autoethanogenum and Incorporation of the Non-naturally Occurring Protein into Microorganisms for the Enhanced Production of CBGA

In a one embodiment the microorganism is *Clostridium autoethanogenum* and it is modified using genetic engineering tools to insert SynNphB-M1 identified by SEQ ID_NO 2.

In a one embodiment the microorganism is *Clostridium autoethanogenum* and it is modified using genetic engineering tools to insert SynNphB-M2 identified by SEQ ID_NO 3.

In a one embodiment the microorganism is *Clostridium autoethanogenum* and it is modified using genetic engineering tools to insert SynNphB-M3 identified by SEQ ID_NO 4.

In a one embodiment the microorganism is *Clostridium autoethanogenum* and it is modified using genetic engineering tools to insert SynNphB-M4 identified by SEQ ID_NO 5.

In a one embodiment the microorganism is *Clostridium autoethanogenum* and it is modified using genetic engineering tools to insert SynNphB-M5 identified by SEQ ID_NO 6.

In a preferred embodiment the microorganism is *Clostridium autoethanogenum* and it is modified using genetic engineering tools to insert SynNphB-M6 identified by SEQ ID_NO 7.

### Genetic Modification of Clostridium autoethanogenum Using Conjugation

In an embodiment, plasmids containing the genes are transformed into an *Escherichia coli* donor strain and then transferred to *Clostridium autoethanogenum* by conjugation. Thiamphenicol (7.5 µg/ml) is used to counter select against the *Escherichia coli* donor strain (Liew et al. 2017).

### Genome Modification of Clostridium autoethanogenum Using CRISPR-Cas System

In an embodiment, *Clostridium autoethanogenum* can be modified genetically via the CRISPR-Cas system (Nagaraju et. al. 2016). This technique enables deletions, insertions or point mutations in the genomic DNA of the microorganism. The system is able to recognize a protospacer adjacent motif (PAM) next to the genomic target site and generate a double-strand break. This is followed by DNA repair initiation through homologous directed repair if an exogenous homologous donor sequence is present.

In an embodiment, *Closteridium autoethanogenum* can be modified genetically via the CRISPR-Cas system. Plasmids containing a Cas protein and a corresponding guide RNA are transformed together with the gene sequence flanked with the DNA fragments complementary to the insertion site. The plasmids are transformed by electroporation and subsequently spread on agar plates, which have been supplemented with antibiotics. By colony PCR, the target sequence is amplified with the help of the corresponding primers and individual positive colonies are then identified with agarose gel electrophoresis and sequencing.

### g. Verification of Transformation

In an embodiment, the shuttle vectors with the incorporated genes are transformed into the microorganism via electroporation and subsequently plated on agar plates containing one or more of the following antibiotics ampicillin, kanamycin, chloramphenicol, tetracycline and spectinomycin. Detection of the successful transformation of gene cassettes is accomplished via PCR and sequencing. The gene transcription and enzyme translation and activity are determined by transcriptome, proteome and metabolome analysis.

### 6. Media Supplementation

The production of CBGA is ensured by the addition of the OA (olivetolic acid) and GPP (geranylpyrophosphate) precursor molecules into the culture medium.

In an embodiment, both OA (olivetolic acid) and GPP (geranylpyrophosphate) are fed to the culture medium at a final concentration between 0.1 g/L and 6 g/L. OA (olivetolic acid) and GPP (geranylpyrophosphate) can be added at the time of inoculation between 1 hour and 18 hours after inoculation.

### 7. Confirmation of CBGA in Solution

The genetically modified microorganism (*Escherichia coli, Zymomonas mobilis, Saccharomyces cerevisiae or Clostridium autoethanogenum*) is cultivated and grown in cell medium.

In an embodiment the cells are disrupted and the precursor molecules OA (olivetolic acid) and GPP (geranylpyrophosphate) are added to the disrupted cell medium. The disrupted cells are incubated together with OA (olivetolic acid) and GPP (geranylpyrophosphate) for 4 hours at 37°C for the enzymatic transformation to CBGA.

In another embodiment a whole cell approach is used where undisrupted cells are cultivated in culture medium and the precursor molecules naringenin are added to the culture medium. The precursor molecules are absorbed by the genetically modified microorganisms and enzymatically transformed into CBGA, which again is released from the genetically modified microorganisms into the culture medium. The cells therefore do not have to be disrupted and the product CBGA is detected in culture medium.

Identification and quantification of CBGA can be performed by several methods combining chromatographic separation like LC, HPLC, UHPLC or GC and mass sensitive or photo optical detection. Chromatographic methods can include liquid (MeOH, ACN, hexan, water, acetic acid and others) or gaseous mobile phases (H₂, He, N₂, Ar) and liquid or solid stationary phases including silica gel, polydimethylsiloxane or reversed phase materials.

Detection of CBGA can be achieved by using MS or MS/MS including sector mass spectrometry, time-of-flight mass spectrometry, the use of quadrupole mass analyzer, three-dimensional quadrupole ion trap, cylindrical ion trap, linear quadrupole ion trap, orbitrap or fourier transform ion cyclotron resonance and the use of an andequat detector, including electron multiplier systems, faraday cups, ion-to-photon detectors, microchannel plate detectors or inductive detectors. Other detection methods like UV-absorption, fluorescence, charged aerosol detector, evaporative light scattering detector, flame ionization detector, flame photometric detector, nitrogen phosphorus detector, atomic-emission detector, refractive index detector, radio flow detector, conductivity monitor, thermal conductivity detector, electron capture detector and photoionization detectors or combination of those principles can be applied.

CBGA can also be detected by using chemical reactions, the use of appropriate stains like iodine vapor, iodoplatinate, marquis reagent, nihydrin, HNO₃-atmosphere, NNCD-reagent, PDAB-TS, TACOT, TCBI, vanillin reagents, Van Urk reagent or xanthydrol and the use of an authentic reference substance (Barker et al. 2012; Mulga et al. 2012).

Quantification can be achieved by the use of an internal standard containing apigenin. By the addition of a known amount of these substances prior to sample preparation it is possible to calculate the actual amount of apigenin and related compounds in a given sample (Barker et al. 2013).

### SEQUENCES

SEQ ID_NO 1:

MSGAAEVERVYSAMEESAGLLDVACSREKIQPILTAFQDVLADGVIVFSMANGRHATE
LDFSISVPAGHGDPYAAALEHGLIPATGHPVGDLLADTQKALPVSMFAVDGEVTSGFK
KTYAFFPTDDMPGLAQLIDIPSMPPSVAENAELFGRYGLDKVQMISLDYKKNQVNLYFS
NLNPEFLQPEPVQAMVREMGLQLPADKGLAFAKRSFAVYPTLSWDSAKIERLCFAVIS
TDPTLAPAQEQADLDLFSTYANNAPYAYAGEKRTLVYGLTLSPSEEYYKLGSYYQISDI
QRKLLKAFDALTD

SEQ ID_NO 2:

MSGAAEVERVYSAMEESAGLLDVACSREKIQPILTAFQDVLADGVIVFSMANGRHATEL
DFSISVPAGHGDPYAAALEHGLIPATGHPVGDLLADTQKALPVSMFAVDGEVTSGFKKT
YAFFPTDDMPGLAQLIDIPSMPPSVAENAELFGRYGLDKVQMISLDYKKNQVNLYFSNL
NPEFLQPEPVQAMVREMGLQLPADKGLAFAKRSFAVYPTLSWDSAKIERLCFAVISTDP
TLAPAQEQADLDLFSTYANNAPYAYAGEKRTLVYGLTLSPSEEYYKLSSAYQISDIQRKL
LKAF

SEQ ID_NO 3:

MSGAAEVERVYSAMEESAGLLDVACSREKIQPILTAFQDVLADGVIVFSMANGRHATE
LDFSISVPAGHGDPYAAALEHGLIPATGHPVGDLLADTQKALPVSMFAVDGEVTSGFK
KTYAFFPTDDMPGLAQLIDIPSMPPSVAENAELFGRYGLDKVQMISLDYKKNQVNLYFS
NLNPEFLQPEPVQAMVREMGLQLPADKGLAFAKRSFAVYPTLSWDSAKIERLCFSVIS
TDPTLAPAQEQADLDLFSTYANNAPYAYAGEKRTLVYGLTLSPSEEYYKLGSAYQISDI
QRKLLKAFDALTD

SEQ ID_NO 4:

MSGAAEVERVYSAMEESAGLLDVACSREKIQPILTAFQDVLADGVIVFSMANGRHATEL
DFSISVPAGHGDPYAAALEHGLIPATGHPVGDLLADTQKALPVSMFAVYGEVTSGFKKT
YAFFPTDDMPGLAQLIDIPSMPPSVAENAELFGRYGLDKVQMISLDYKKNQVNLYFSNL
NPEFLQPEPVQAMVREMGLQLPADKGLAFAKRSFAVYPTLSWDSAKIERLCFAVISTDP
TLAPAQEQADLDLFSTYANNAPYAYAGEKRTLVYGLTLSPSEEYYKLSSAYQISDIQRKL
LKAFDALTD

SEQ ID_NO 5:

MSGAAEVERVYSAMEESAGLLDVACSREKIQPILTAFQDVLADGVIVFSMANGRHATEL
DFSISVPAGHGDPYAAALEHGLIPATGHPVGDLLADTQKALPVSMFAVLGEVTSGFKKT
YAFFPTDDMPGLAQLIDIPSMPPSVAENAELFGRYGLDKVQMISLDYKKNQVNLYFSNL
NPEFLQPEPVQAMVREMGLQLPADKGLAFAKRSFAVYPTLSWDSAKIERLCFAVISTDP
TLAPAQEQADLDLFSTYANNAPYAYAGEKRTLVYGLTLSPSEEYYKLSSAYQISDIQRKL
LKAFDALTD

SEQ ID_NO 6:

MSGAAEVERVYSAMEESAGLLDVACSREKIQPILTAFQDVLADGVIVFSMANGRHATEL
D

FSISVPAGHGDPYAAALEHGLIPATGHPVGDLLADTQKALPVSMFAVIGEVTSGFKKTY
A

FFPTDDMPGLAQLIDIPSMPPSVAENAELFGRYGLDKVQMISLDYKKNQVNLYFSNLNP
E

FLQPEPVQAMVREMGLQLPADKGLAFAKRSFAVYPTLSWDSAKIERLCFAVISTDPTLA
P

AQEQADLDLFSTYANNAPYAYAGEKRTLVYGLTLSPSEEYYKLSSAYQISDIQRKLLKAF

DALTD

SEQ ID_NO 7:

MSGAAEVERVYSAMEESAGLLDVACSREKIQPILTAFQDVLADGVIVFSMANGRHATEL
D

FSISVPAGHGDPYAAALEHGLIPATGHPVGDLLADTQKALPVSMFAVNGEVTSGFKKTY
A

FFPTDDMPGLAQLIDIPSMPPSVAENAELFGRYGLDKVQMISLDYKKNQVNLYFSNLNP
E

FLQPEPVQAMVREMGLQLPADKGLAFAKRSFAVYPTLSWDSAKIERLCFAVISTDPTLA
P

AQEQADLDLFSTYANNAPYAYAGEKRTLVYGLTLSPSEEYYKLSSAYQISDIQRKLLKAF

DALTD

### Figure Description

Figure 1: NphB catalysis the C-C bond formation between geranyl pyrophosphate and olivetoric acid to form cannabigerolic acid.
Figure 2: Enzymatic synthesis of different cannabinoids based on CBGA as precursor.

## Claims

1. A non-naturally occurring protein sequence able to enhance the production of CBGA in microorganisms while decreasing the yield of the side-production 2-O-geranyl olivetolate.

2. A non-naturally occurring protein sequence according to claim 1, wherein the protein sequence functions as an enhanced prenyltransferase.

3. A non-naturally occurring protein sequence according to claim 1, wherein the sequence is SEQ ID_NO 2 or has at least a 70% identity with SEQ ID_NO 2, or has at least an 80% with SEQ ID_NO 2, or has at least a 90% with SEQ ID_NO 2.

4. A non-naturally occurring protein sequence according to claim 1, wherein the sequence is SEQ ID_NO 3 or has at least a 70% identity with SEQ ID_NO 3, or has at least an 80% with SEQ ID_NO 3, or has at least a 90% with SEQ ID_NO 3.

5. A non-naturally occurring protein sequence according to claim 1, wherein the sequence is SEQ ID_NO 4 or has at least a 70% identity with SEQ ID_NO 4, or has at least an 80% with SEQ ID_NO 4, or has at least a 90% with SEQ ID_NO 4.

6. A non-naturally occurring protein sequence according to claim 1, wherein the sequence is SEQ ID_NO 5 or has at least a 70% identity with SEQ ID_NO 5, or has at least an 80% with SEQ ID_NO 5, or has at least a 90% with SEQ ID_NO 5.

7. A non-naturally occurring protein sequence according to claim 1, wherein the sequence is SEQ ID_NO 6, or has at least a 70% identity with SEQ ID_NO 6, or has at least an 80% with SEQ ID_NO 6, or has at least a 90% with SEQ ID_NO 6.

8. A non-naturally occurring protein sequence according to claim 1, wherein the sequence is SEQ ID_NO 7, or has at least a 70% identity with SEQ ID_NO 7, or has at least an 80% with SEQ ID_NO 7, or has at least a 90% with SEQ ID_NO 7.

9. A non-naturally occurring protein sequence according to claim 1, wherein the microorganism is *Escherichia coli.*

10. A non-naturally occurring protein sequence according to claim 27, wherein the microorganism is *Escherichia coli* and it is selected from *Escherichia coli BLR (DE3), Escherichia coli Rosetta2 (DE3), Escherichia coli T7 Express* and *Escherichia coli BL21 (DE3).*

11. A non-naturally occurring protein sequence according to any of claims 1 to 8, wherein the microorganism is *Zymomonas mobilis* ZM4.

12. A non-naturally occurring protein sequence according to any of claims 1 to 8, wherein the microorganism is *Saccharomyces Cerevisiae.*

13. A non-naturally occurring protein sequence according to any of claims 1 to 8, wherein the microorganism is *Clostridium autoethanogenum.*

14. A method of producing a non-naturally occurring protein sequence able to enhance the production of CBGA in microorganisms while decreasing the yield of the side-production 2-O-geranyl olivetolate.

15. A method of modifying microorganisms with a non-naturally occurring protein sequence able to enhance the production of CBGA in microorganisms while decreasing the yield of the side-production 2-O-geranyl olivetolate.


SEQUENCE LISTING

<110> Synbionik Gmbh

<120> Optimized production of CBGA from Olivetol acid and geranyl
pyrophosphate via SynNphB

<130> Proteins for Enhanced Production of CBGA

<160> 7

<170> BiSSAP 1.3.6

<210> 1
<211> 305
<212> PRT
<213> Streptomyces sp.


<220>
<223> Wild Type Sequence - WP_078582630.1 GI: 1154209950

<400> 1
Met Ser Gly Ala Ala Glu Val Glu Arg Val Tyr Ser Ala Met Glu Glu
1               5                   10                  15
Ser Ala Gly Leu Leu Asp Val Ala Cys Ser Arg Glu Lys Ile Gln Pro
            20                  25                  30
Ile Leu Thr Ala Phe Gln Asp Val Leu Ala Asp Gly Val Ile Val Phe
        35                  40                  45
Ser Met Ala Asn Gly Arg His Ala Thr Glu Leu Asp Phe Ser Ile Ser
    50                  55                  60
Val Pro Ala Gly His Gly Asp Pro Tyr Ala Ala Ala Leu Glu His Gly
65                  70                  75                  80
Leu Ile Pro Ala Thr Gly His Pro Val Gly Asp Leu Leu Ala Asp Thr
                85                  90                  95
Gln Lys Ala Leu Pro Val Ser Met Phe Ala Val Asp Gly Glu Val Thr
            100                 105                 110
Ser Gly Phe Lys Lys Thr Tyr Ala Phe Phe Pro Thr Asp Asp Met Pro
        115                 120                 125
Gly Leu Ala Gln Leu Ile Asp Ile Pro Ser Met Pro Pro Ser Val Ala
    130                 135                 140
Glu Asn Ala Glu Leu Phe Gly Arg Tyr Gly Leu Asp Lys Val Gln Met
145                 150                 155                 160
Ile Ser Leu Asp Tyr Lys Lys Asn Gln Val Asn Leu Tyr Phe Ser Asn
                165                 170                 175
Leu Asn Pro Glu Phe Leu Gln Pro Glu Pro Val Gln Ala Met Val Arg
            180                 185                 190
Glu Met Gly Leu Gln Leu Pro Ala Asp Lys Gly Leu Ala Phe Ala Lys
        195                 200                 205
Arg Ser Phe Ala Val Tyr Pro Thr Leu Ser Trp Asp Ser Ala Lys Ile
    210                 215                 220
Glu Arg Leu Cys Phe Ala Val Ile Ser Thr Asp Pro Thr Leu Ala Pro
225                 230                 235                 240
Ala Gln Glu Gln Ala Asp Leu Asp Leu Phe Ser Thr Tyr Ala Asn Asn
                245                 250                 255
Ala Pro Tyr Ala Tyr Ala Gly Glu Lys Arg Thr Leu Val Tyr Gly Leu
            260                 265                 270
Thr Leu Ser Pro Ser Glu Glu Tyr Tyr Lys Leu Gly Ser Tyr Tyr Gln
        275                 280                 285
Ile Ser Asp Ile Gln Arg Lys Leu Leu Lys Ala Phe Asp Ala Leu Thr
    290                 295                 300
Asp
305

<210> 2
<211> 305
<212> PRT
<213> Artificial Sequence

<220>
<223> SynNphB-M1 - (G284S) (Y286A)

<400> 2
Met Ser Gly Ala Ala Glu Val Glu Arg Val Tyr Ser Ala Met Glu Glu
1               5                   10                  15
Ser Ala Gly Leu Leu Asp Val Ala Cys Ser Arg Glu Lys Ile Gln Pro
            20                  25                  30
Ile Leu Thr Ala Phe Gln Asp Val Leu Ala Asp Gly Val Ile Val Phe
        35                  40                  45
Ser Met Ala Asn Gly Arg His Ala Thr Glu Leu Asp Phe Ser Ile Ser
    50                  55                  60
Val Pro Ala Gly His Gly Asp Pro Tyr Ala Ala Ala Leu Glu His Gly
65                  70                  75                  80
Leu Ile Pro Ala Thr Gly His Pro Val Gly Asp Leu Leu Ala Asp Thr
                85                  90                  95
Gln Lys Ala Leu Pro Val Ser Met Phe Ala Val Asp Gly Glu Val Thr
            100                 105                 110
Ser Gly Phe Lys Lys Thr Tyr Ala Phe Phe Pro Thr Asp Asp Met Pro
        115                 120                 125
Gly Leu Ala Gln Leu Ile Asp Ile Pro Ser Met Pro Pro Ser Val Ala
    130                 135                 140
Glu Asn Ala Glu Leu Phe Gly Arg Tyr Gly Leu Asp Lys Val Gln Met
145                 150                 155                 160
Ile Ser Leu Asp Tyr Lys Lys Asn Gln Val Asn Leu Tyr Phe Ser Asn
                165                 170                 175
Leu Asn Pro Glu Phe Leu Gln Pro Glu Pro Val Gln Ala Met Val Arg
            180                 185                 190
Glu Met Gly Leu Gln Leu Pro Ala Asp Lys Gly Leu Ala Phe Ala Lys
        195                 200                 205
Arg Ser Phe Ala Val Tyr Pro Thr Leu Ser Trp Asp Ser Ala Lys Ile
    210                 215                 220
Glu Arg Leu Cys Phe Ala Val Ile Ser Thr Asp Pro Thr Leu Ala Pro
225                 230                 235                 240
Ala Gln Glu Gln Ala Asp Leu Asp Leu Phe Ser Thr Tyr Ala Asn Asn
                245                 250                 255
Ala Pro Tyr Ala Tyr Ala Gly Glu Lys Arg Thr Leu Val Tyr Gly Leu
            260                 265                 270
Thr Leu Ser Pro Ser Glu Glu Tyr Tyr Lys Leu Ser Ser Ala Tyr Gln
        275                 280                 285
Ile Ser Asp Ile Gln Arg Lys Leu Leu Lys Ala Phe Asp Ala Leu Thr
    290                 295                 300
Asp
305

<210> 3
<211> 305
<212> PRT
<213> Artificial Sequence

<220>
<223> SynNphB-M2 - (A230S) (Y286A)

<400> 3
Met Ser Gly Ala Ala Glu Val Glu Arg Val Tyr Ser Ala Met Glu Glu

1               5                   10                  15
Ser Ala Gly Leu Leu Asp Val Ala Cys Ser Arg Glu Lys Ile Gln Pro
            20                  25                  30
Ile Leu Thr Ala Phe Gln Asp Val Leu Ala Asp Gly Val Ile Val Phe
        35                  40                  45
Ser Met Ala Asn Gly Arg His Ala Thr Glu Leu Asp Phe Ser Ile Ser
    50                  55                  60
Val Pro Ala Gly His Gly Asp Pro Tyr Ala Ala Ala Leu Glu His Gly
65                  70                  75                  80
Leu Ile Pro Ala Thr Gly His Pro Val Gly Asp Leu Leu Ala Asp Thr
                85                  90                  95
Gln Lys Ala Leu Pro Val Ser Met Phe Ala Val Asp Gly Glu Val Thr
            100                 105                 110
Ser Gly Phe Lys Lys Thr Tyr Ala Phe Phe Pro Thr Asp Asp Met Pro
        115                 120                 125
Gly Leu Ala Gln Leu Ile Asp Ile Pro Ser Met Pro Pro Ser Val Ala
    130                 135                 140
Glu Asn Ala Glu Leu Phe Gly Arg Tyr Gly Leu Asp Lys Val Gln Met
145                 150                 155                 160
Ile Ser Leu Asp Tyr Lys Lys Asn Gln Val Asn Leu Tyr Phe Ser Asn
                165                 170                 175
Leu Asn Pro Glu Phe Leu Gln Pro Glu Pro Val Gln Ala Met Val Arg
            180                 185                 190
Glu Met Gly Leu Gln Leu Pro Ala Asp Lys Gly Leu Ala Phe Ala Lys
        195                 200                 205
Arg Ser Phe Ala Val Tyr Pro Thr Leu Ser Trp Asp Ser Ala Lys Ile
    210                 215                 220
Glu Arg Leu Cys Phe Ser Val Ile Ser Thr Asp Pro Thr Leu Ala Pro
225                 230                 235                 240
Ala Gln Glu Gln Ala Asp Leu Asp Leu Phe Ser Thr Tyr Ala Asn Asn
                245                 250                 255
Ala Pro Tyr Ala Tyr Ala Gly Glu Lys Arg Thr Leu Val Tyr Gly Leu
            260                 265                 270
Thr Leu Ser Pro Ser Glu Glu Tyr Tyr Lys Leu Gly Ser Ala Tyr Gln
        275                 280                 285
Ile Ser Asp Ile Gln Arg Lys Leu Leu Lys Ala Phe Asp Ala Leu Thr
    290                 295                 300
Asp
305

<210> 4
<211> 305
<212> PRT
<213> Artificial Sequence

<220>
<223> SynNphB-M3 - (D108Y) (G284S) (Y286A)

<400> 4
Met Ser Gly Ala Ala Glu Val Glu Arg Val Tyr Ser Ala Met Glu Glu
1               5                   10                  15
Ser Ala Gly Leu Leu Asp Val Ala Cys Ser Arg Glu Lys Ile Gln Pro
            20                  25                  30
Ile Leu Thr Ala Phe Gln Asp Val Leu Ala Asp Gly Val Ile Val Phe
        35                  40                  45
Ser Met Ala Asn Gly Arg His Ala Thr Glu Leu Asp Phe Ser Ile Ser
    50                  55                  60
Val Pro Ala Gly His Gly Asp Pro Tyr Ala Ala Ala Leu Glu His Gly
65                  70                  75                  80
Leu Ile Pro Ala Thr Gly His Pro Val Gly Asp Leu Leu Ala Asp Thr
                85                  90                  95
Gln Lys Ala Leu Pro Val Ser Met Phe Ala Val Tyr Gly Glu Val Thr

            100                 105                 110
Ser Gly Phe Lys Lys Thr Tyr Ala Phe Phe Pro Thr Asp Asp Met Pro
        115                 120                 125
Gly Leu Ala Gln Leu Ile Asp Ile Pro Ser Met Pro Pro Ser Val Ala
    130                 135                 140
Glu Asn Ala Glu Leu Phe Gly Arg Tyr Gly Leu Asp Lys Val Gln Met
145                 150                 155                 160
Ile Ser Leu Asp Tyr Lys Lys Asn Gln Val Asn Leu Tyr Phe Ser Asn
                165                 170                 175
Leu Asn Pro Glu Phe Leu Gln Pro Glu Pro Val Gln Ala Met Val Arg
            180                 185                 190
Glu Met Gly Leu Gln Leu Pro Ala Asp Lys Gly Leu Ala Phe Ala Lys
        195                 200                 205
Arg Ser Phe Ala Val Tyr Pro Thr Leu Ser Trp Asp Ser Ala Lys Ile
    210                 215                 220
Glu Arg Leu Cys Phe Ala Val Ile Ser Thr Asp Pro Thr Leu Ala Pro
225                 230                 235                 240
Ala Gln Glu Gln Ala Asp Leu Asp Leu Phe Ser Thr Tyr Ala Asn Asn
                245                 250                 255
Ala Pro Tyr Ala Tyr Ala Gly Glu Lys Arg Thr Leu Val Tyr Gly Leu
            260                 265                 270
Thr Leu Ser Pro Ser Glu Glu Tyr Tyr Lys Leu Ser Ser Ala Tyr Gln
        275                 280                 285
Ile Ser Asp Ile Gln Arg Lys Leu Leu Lys Ala Phe Asp Ala Leu Thr
    290                 295                 300
Asp
305

<210> 5
<211> 305
<212> PRT
<213> Artificial Sequence

<220>
<223> SynNphB-M4 - (D108L)(G284S) (Y286A)

<400> 5
Met Ser Gly Ala Ala Glu Val Glu Arg Val Tyr Ser Ala Met Glu Glu
1               5                   10                  15
Ser Ala Gly Leu Leu Asp Val Ala Cys Ser Arg Glu Lys Ile Gln Pro
            20                  25                  30
Ile Leu Thr Ala Phe Gln Asp Val Leu Ala Asp Gly Val Ile Val Phe
        35                  40                  45
Ser Met Ala Asn Gly Arg His Ala Thr Glu Leu Asp Phe Ser Ile Ser
    50                  55                  60
Val Pro Ala Gly His Gly Asp Pro Tyr Ala Ala Ala Leu Glu His Gly
65                  70                  75                  80
Leu Ile Pro Ala Thr Gly His Pro Val Gly Asp Leu Leu Ala Asp Thr
                85                  90                  95
Gln Lys Ala Leu Pro Val Ser Met Phe Ala Val Leu Gly Glu Val Thr
            100                 105                 110
Ser Gly Phe Lys Lys Thr Tyr Ala Phe Phe Pro Thr Asp Asp Met Pro
        115                 120                 125
Gly Leu Ala Gln Leu Ile Asp Ile Pro Ser Met Pro Pro Ser Val Ala
    130                 135                 140
Glu Asn Ala Glu Leu Phe Gly Arg Tyr Gly Leu Asp Lys Val Gln Met
145                 150                 155                 160
Ile Ser Leu Asp Tyr Lys Lys Asn Gln Val Asn Leu Tyr Phe Ser Asn
                165                 170                 175
Leu Asn Pro Glu Phe Leu Gln Pro Glu Pro Val Gln Ala Met Val Arg
            180                 185                 190
Glu Met Gly Leu Gln Leu Pro Ala Asp Lys Gly Leu Ala Phe Ala Lys

195                 200                 205
Arg Ser Phe Ala Val Tyr Pro Thr Leu Ser Trp Asp Ser Ala Lys Ile
    210                 215                 220
Glu Arg Leu Cys Phe Ala Val Ile Ser Thr Asp Pro Thr Leu Ala Pro
225                 230                 235                 240
Ala Gln Glu Gln Ala Asp Leu Asp Leu Phe Ser Thr Tyr Ala Asn Asn
                245                 250                 255
Ala Pro Tyr Ala Tyr Ala Gly Glu Lys Arg Thr Leu Val Tyr Gly Leu
            260                 265                 270
Thr Leu Ser Pro Ser Glu Glu Tyr Tyr Lys Leu Ser Ser Ala Tyr Gln
        275                 280                 285
Ile Ser Asp Ile Gln Arg Lys Leu Leu Lys Ala Phe Asp Ala Leu Thr
    290                 295                 300
Asp
305

<210> 6
<211> 305
<212> PRT
<213> Artificial Sequence

<220>
<223> SynNphB-M5 - (D108I) (G284S) (Y286A)

<400> 6
Met Ser Gly Ala Ala Glu Val Glu Arg Val Tyr Ser Ala Met Glu Glu
1               5                   10                  15
Ser Ala Gly Leu Leu Asp Val Ala Cys Ser Arg Glu Lys Ile Gln Pro
            20                  25                  30
Ile Leu Thr Ala Phe Gln Asp Val Leu Ala Asp Gly Val Ile Val Phe
        35                  40                  45
Ser Met Ala Asn Gly Arg His Ala Thr Glu Leu Asp Phe Ser Ile Ser
    50                  55                  60
Val Pro Ala Gly His Gly Asp Pro Tyr Ala Ala Ala Leu Glu His Gly
65                  70                  75                  80
Leu Ile Pro Ala Thr Gly His Pro Val Gly Asp Leu Leu Ala Asp Thr
                85                  90                  95
Gln Lys Ala Leu Pro Val Ser Met Phe Ala Val Ile Gly Glu Val Thr
            100                 105                 110
Ser Gly Phe Lys Lys Thr Tyr Ala Phe Phe Pro Thr Asp Asp Met Pro
        115                 120                 125
Gly Leu Ala Gln Leu Ile Asp Ile Pro Ser Met Pro Pro Ser Val Ala
    130                 135                 140
Glu Asn Ala Glu Leu Phe Gly Arg Tyr Gly Leu Asp Lys Val Gln Met
145                 150                 155                 160
Ile Ser Leu Asp Tyr Lys Lys Asn Gln Val Asn Leu Tyr Phe Ser Asn
                165                 170                 175
Leu Asn Pro Glu Phe Leu Gln Pro Glu Pro Val Gln Ala Met Val Arg
            180                 185                 190
Glu Met Gly Leu Gln Leu Pro Ala Asp Lys Gly Leu Ala Phe Ala Lys
        195                 200                 205
Arg Ser Phe Ala Val Tyr Pro Thr Leu Ser Trp Asp Ser Ala Lys Ile
    210                 215                 220
Glu Arg Leu Cys Phe Ala Val Ile Ser Thr Asp Pro Thr Leu Ala Pro
225                 230                 235                 240
Ala Gln Glu Gln Ala Asp Leu Asp Leu Phe Ser Thr Tyr Ala Asn Asn
                245                 250                 255
Ala Pro Tyr Ala Tyr Ala Gly Glu Lys Arg Thr Leu Val Tyr Gly Leu
            260                 265                 270
Thr Leu Ser Pro Ser Glu Glu Tyr Tyr Lys Leu Ser Ser Ala Tyr Gln
        275                 280                 285
Ile Ser Asp Ile Gln Arg Lys Leu Leu Lys Ala Phe Asp Ala Leu Thr

290                 295                 300
Asp
305

<210> 7
<211> 305
<212> PRT
<213> Artificial Sequence


<220>
<223> SynNphB-M6 - (D108N) (G284S) (Y286A)

<400> 7
Met Ser Gly Ala Ala Glu Val Glu Arg Val Tyr Ser Ala Met Glu Glu
1               5                   10                  15
Ser Ala Gly Leu Leu Asp Val Ala Cys Ser Arg Glu Lys Ile Gln Pro
            20                  25                  30
Ile Leu Thr Ala Phe Gln Asp Val Leu Ala Asp Gly Val Ile Val Phe
        35                  40                  45
Ser Met Ala Asn Gly Arg His Ala Thr Glu Leu Asp Phe Ser Ile Ser
    50                  55                  60
Val Pro Ala Gly His Gly Asp Pro Tyr Ala Ala Ala Leu Glu His Gly
65                  70                  75                  80
Leu Ile Pro Ala Thr Gly His Pro Val Gly Asp Leu Leu Ala Asp Thr
                85                  90                  95
Gln Lys Ala Leu Pro Val Ser Met Phe Ala Val Asn Gly Glu Val Thr
            100                 105                 110
Ser Gly Phe Lys Lys Thr Tyr Ala Phe Phe Pro Thr Asp Asp Met Pro
        115                 120                 125
Gly Leu Ala Gln Leu Ile Asp Ile Pro Ser Met Pro Pro Ser Val Ala
    130                 135                 140
Glu Asn Ala Glu Leu Phe Gly Arg Tyr Gly Leu Asp Lys Val Gln Met
145                 150                 155                 160
Ile Ser Leu Asp Tyr Lys Lys Asn Gln Val Asn Leu Tyr Phe Ser Asn
                165                 170                 175
Leu Asn Pro Glu Phe Leu Gln Pro Glu Pro Val Gln Ala Met Val Arg
            180                 185                 190
Glu Met Gly Leu Gln Leu Pro Ala Asp Lys Gly Leu Ala Phe Ala Lys
        195                 200                 205
Arg Ser Phe Ala Val Tyr Pro Thr Leu Ser Trp Asp Ser Ala Lys Ile
    210                 215                 220
Glu Arg Leu Cys Phe Ala Val Ile Ser Thr Asp Pro Thr Leu Ala Pro
225                 230                 235                 240
Ala Gln Glu Gln Ala Asp Leu Asp Leu Phe Ser Thr Tyr Ala Asn Asn
                245                 250                 255
Ala Pro Tyr Ala Tyr Ala Gly Glu Lys Arg Thr Leu Val Tyr Gly Leu
            260                 265                 270
Thr Leu Ser Pro Ser Glu Glu Tyr Tyr Lys Leu Ser Ser Ala Tyr Gln
        275                 280                 285
Ile Ser Asp Ile Gln Arg Lys Leu Leu Lys Ala Phe Asp Ala Leu Thr
    290                 295                 300
Asp
305